# EUROPEAN PATENT APPLICATION

(11) **EP 3 708 648 A1**
(43) Date of publication of application: **16.09.2020**
(21) Application number: 18876536.6
(22) Date of filing: 08.11.2018
(51) Int. Cl.: C12M 3/00, C09D 185/02, C09D 201/06

(54) **CELL CULTURE VESSEL**

(30) Priority: 10.11.2017 JP 2017217033; 14.03.2018 JP 2018046777
(71) Applicant: Nissan Chemical Corporation, Tokyo 103-6119 (JP)
(72) Inventor: NAKAJIMA Hiroyuki, Shiraoka-shi Saitama 349-0294 (JP); NISHINO Taito, Shiraoka-shi Saitama 349-0294 (JP); HIROI Yoshiomi, Funabashi-shi Chiba 274-0052 (JP); SUZUKI Kohei, Funabashi-shi Chiba 274-0052 (JP)
(74) Representative: dompatent von Kreisler Selting Werner - Partnerschaft von Patent- und Rechtsanwälten mbB
(86) International application number: PCT/JP2018/041556
(87) International publication number: WO 2019/093442

(57) **Abstract**

Provide is a cell culture vessel in which adhesion of a peptide relating to cell proliferation is inhibited.

Provide is a cell culture vessel which comprises a coating which inhibits adhesion of a peptide relating to cell proliferation at least a part of the surface thereof, in particular a coating containing a recurring unit which contains a group represented by the following formula (a) and a recurring unit which contains a group represented by the following formula (b). 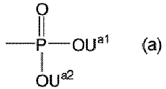 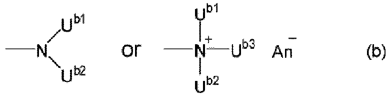 (wherein, U^{a1}, U^{a2}, U^{a3}, U^{b1}, U^{b2}, U^{b3} and An⁻ are as defined in the present specification and Claims.)

## Description

### TECHNICAL FIELD

The present invention relates to a cell culture vessel having a coating which inhibits adhesion of peptides relating to cell proliferation.

### BACKGROUND ART

In recent years, technologies to proliferate or maintain various organs, tissues and cells that play different roles in animals and plants *in vitro* have been developed. To proliferate or maintain these organs and tissues *in vitro* is called organ culture and tissue culture, respectively, and to proliferate, differentiate or maintain cells separated from organs and tissues *in vitro* is called cell culture. The cell culture is a technology for proliferating, differentiating or maintaining isolated cells in a medium *in vitro,* and is indispensable for analyzing functions and structures of various organs, tissues and cells *in vivo* in detail. In addition, the cells and/or tissues cultured by the technology are utilized in various fields such as medical efficacy and toxicity evaluation of chemical substances, pharmaceuticals, etc., mass production of useful substances such as enzymes, cell growth factors, antibodies, etc., regenerative medicine to supplement organs, tissues and cells lost by diseases or defects, selective breeding of plants, creation of genetically recombined crops, and the like.

For culture of cells, cell culture vessels such as schale (petri dishes), dishes, flasks, bags, plates, chamber slides, tubes, trays, bottles, etc., are generally used, and are appropriately selected depending on various conditions such as a kind of cells, culture method, culture scale, etc. The material of cell culture vessel is exclusively glass or a resin and, for example, when the cell culture vessel is made of a resin and the surface thereof is hydrophobic, there is a problem that adhesion of cells and components indispensable for cell culture such as cell growth factors, etc., to the surface of the vessel occurs whereby proliferatability and expression of functions of cells are impaired.

In order to solve such a problem, it has heretofore been proposed a cell culture vessel in which the surface thereof is modified to hydrophilic by plasma treatment or corona discharge treatment, or coating treatment of a hydrophilic compound. However, in the treatment of the coating treatment of the hydrophilic compound, the hydrophilic compound is eluted from the coating applied to the vessel into the culture medium, so that not only causing lowering in adhesion inhibiting ability of cells and components indispensable for cell culture, but also contaminating the culture medium, whereby there is a fear that bad effects are exerted to cells and components indispensable for cell culture, and there is a problem that impurities remain in final products in regenerative medicine or cell medicine. In addition, there has been proposed not only modification of the surface of a cell culture vessel, but also a cell culture medium to which a water-soluble synthetic polymer such as polyvinyl alcohol or polyethylene glycol, etc., is added (for example, see Patent Document 1). It has been reported that the water-soluble synthetic polymer added to the cell culture medium is physically adsorbed onto the surface of the cell culture vessel, and adhesion of the cells onto the surface of the vessel and adsorption of the component indispensable for cell culture such as cell growth factors, etc., are inhibited. However, addition of the synthetic polymer to the culture medium similarly contaminate the culture medium, whereby there is a fear that bad effects are exerted to cells and components indispensable for cell culture, and there is a problem that it remains as impurities in final products in regenerative medicine or cell medicine, etc.

The present inventors paid attention to a polymer having a phosphate ester group, which is expected as a coating material having an adhesion inhibiting ability of various biological substances, and carried out in-depth studies. As a result, they have reported that a coating agent containing a copolymer which contains a specific anionic group and a cationic group can firmly adhere irrespective of the kind of the substrate, and after adhesion, it becomes a coating film excellent in resistance to an aqueous solvent, and shows excellent adhesion inhibiting ability to a biological substance (for example, platelets, fibrinogen, etc.) (for example, see Patent Documents 2 and 3). In addition, it has been also reported that a cell culture vessel which comprises coated by the above-mentioned coating agent is excellent in cell-adhesion inhibiting ability and also excellent in resistance to solvents and radiation (for example, see Patent Document 4).

### PRIOR ART DOCUMENTS

### PATENT DOCUMENTS

Patent Document 1: JP 2007-124982A
Patent Document 2: WO 2014/196650
Patent Document 3: WO 2016/093293
Patent Document 4: WO 2014/196652

### SUMMARY OF THE INVENTION

### PROBLEMS TO BE SOLVED BY THE INVENTION

For the coating to be applied to the cell culture vessel, it is desired to have not only cell-adhesion inhibiting ability and resistance to aqueous solvents and radiation but also adhesion inhibiting ability of peptides relating to cell proliferation such as cell growth factors, etc., in the point of proliferation of cells and expression of functions. However, it has not been reported a cell culture vessel provided with a coating having these properties in combination.

### MEANS TO SOLVE THE PROBLEMS

The present inventors have found that a cell culture vessel provided with a coating containing a copolymer which contains a specific anionic structure, a specific cationic structure, and a specific hydrophobic structure according to circumstances, at least a part of the surface of the vessel, has not only excellent cell-adhesion inhibiting ability and resistance to an aqueous solvent or radiation, but also excellent in cell-adhesion inhibiting ability of peptides relating to cell proliferation such as cell growth factors, etc., whereby the present invention has accomplished.

The present invention is as follows.
[1] A cell culture vessel having a coating which inhibits adhesion of a peptide relating to cell proliferation at least a part of the surface thereof.
[2] The cell culture vessel described in the above [1], wherein the peptide relating to cell proliferation is a peptide relating to cell proliferation through a receptor or a signal, or a peptide relating to nutrient transportation.
[3] The cell culture vessel described in the above [2], wherein the peptide relating to cell proliferation through a receptor or a signal is selected from the group consisting of a cell growth factor, a hematopoietic factor, a neurotrophic factor, an interleukin and a hormone.
[4] The cell culture vessel described in the above [1], wherein the coating contains a copolymer containing a recurring unit which contains a group represented by the following formula (a) and a recurring unit which contains a group represented by the following formula (b): 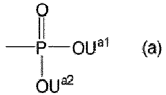 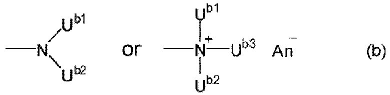 (wherein
   U^{a1}, U^{a2}, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion).
[5] The cell culture vessel described in the above [4], wherein the copolymer further contains a recurring unit which contains a group represented by the following formula (c):

   -R^{c} (c)

   [wherein
   R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (where, the above-mentioned aryl portion may be substituted by a linear or branched alkyl group having 1 to 5 carbon atoms which may be substituted by a halogen atom(s))].
[6] The cell culture vessel described in the above [3], wherein the cell growth factor is selected from the group consisting of an epidermal growth factor (EGF), an insulin-like growth factor (IGF), a transforming growth factor-β (TGF-β), a nerve growth factor (NGF), a brain-derived neurotrophic factor (BDNF), a vascular endothelial cell growth factor (VEGF), a hepatocyte growth factor (HGF), a platelet-derived growth factor (PDGF), an acidic fibroblast cell growth factor (aFGF) and a basic fibroblast cell growth factor (bFGF).
[7] The cell culture vessel described in the above [3], wherein the hematopoietic factor is selected from the group consisting of a granulocyte-colony stimulating factor (G-CSF), a granulocyte-macrophage colony-stimulating factor (GM-CSF), a macrophage colony-stimulating factor (M-CSF), a monocyte chemotactic factor (MCP-1), erythropoietin (EPO) and thrombopoietin (TPO).
[8] The cell culture vessel described in the above [3], wherein the interleukin is selected from the group consisting of interleukin-la, interleukin-1β, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18 and leukemia inhibitory factor (LIF).
[9] The cell culture vessel described in the above [3], wherein the neurotrophic factor is selected from the group consisting of nerve growth factor (NGF), brain-derived neurotrophic factor (BDNF), neurotrophin 3 and neurotrophin 4/5.
[10] The cell culture vessel described in the above [3], wherein the hormone is selected from the group consisting of insulin, glucagon, adrenocorticotropic hormones, thyroid stimulating hormones, growth hormones, follicle stimulating hormones, luteotropic hormones, prolactin and fibronectin.
[11] The cell culture vessel described in the above [2], wherein the peptide relating to nutrient transportation is transferrin or bovine serum albumin (BSA).
[12] A method for increasing a proliferation rate of cells cultured in a cell culture vessel which comprises subjecting a coating which inhibits adhesion of a peptide relating to cell proliferation to at least a part of a surface of the vessel.
[13] A method for reducing an amount of a peptide relating to cell proliferation to be added to a cell culture vessel which comprises subjecting a coating which inhibits adhesion of a peptide relating to cell proliferation to at least a part of a surface of the vessel.

### EFFECTS OF THE INVENTION

The cell culture vessel of the present invention can inhibit adhesion of not only cells but also peptides relating to cell proliferation by being provided with a coating which inhibit adhesion of peptides relating to cell proliferation, in particular, a coating which contains a copolymer containing an anionic group represented by the formula (a), a cationic group represented by the formula (b), and, if necessary, a hydrophobic group represented by the formula (c), at least a part of the surface of the vessel. In addition, the cationic group and the anionic group in the coating form an ionic bond (ion complex) so that the coating can adhere to a substrate material of the vessel without selecting the kind of the material such as glass, fiber, inorganic particles and a resin (synthetic resin and natural resin), etc., and further, after adhesion, it becomes a coating excellent in resistance to an aqueous solvent (water, phosphate-buffered saline (PBS), an alcohol, etc.). That is, according to the present invention, it is possible to provide a cell culture vessel which can inhibit adhesion of peptides relating to cell proliferation to the surface of the vessel in addition to cells, and also has excellent resistance to solvents including a culture medium.

### BRIEF DESCRIPTION OF THE DRAWINGS

Fig. 1 is a graph showing a result of measuring a proliferation rate by culturing SKN cells in the presence or absence of bFGF (0, 0.01, 0.1, 1, 10 and 100 ng/mL) in accordance with Test Example 1 using a microplate coated in Example 1 and a non-coating microplate.
Fig. 2 is a graph showing an ED50 value of a bFGF concentration to a cell proliferation rate in a microplate coated in Example 1 and a non-coating microplate calculated based on the result of Fig. 1.
Fig. 3 is a graph showing a result of measuring a proliferation rate by culturing SKN cells in the presence or absence of bFGF (0, 0.01, 0.1, 1, 10 and 100 ng/mL) in accordance with Test Example 2 using a microplate coated in Example 2 and a non-coating microplate.
Fig. 4 is a graph showing an ED50 value of a bFGF concentration to a cell proliferation rate in a microplate coated in Example 2 and a non-coating microplate calculated based on the result of Fig. 3.
Fig. 5 is a graph showing a result of measuring a proliferation rate by culturing TF1 cells in the presence or absence of IL3 (0, 0.001, 0.01, 0.1, 1 and 10 ng/mL) in accordance with Test Example 3 using a microplate coated in Example 1 and a non-coating microplate.
Fig. 6 is a graph showing an ED50 value of an IL3 concentration to a cell proliferation rate in a microplate coated in Example 1 and a non-coating microplate calculated based on the result of Fig. 5.
Fig. 7 is a graph showing a result of measuring a bFGF amount remained in a medium without adsorbing to a plate in accordance with Test Example 4 using a microplate coated in Example 1 and a non-coating microplate.
Fig. 8 is a graph showing a result of measuring an IL3 amount remained in a medium without adsorbing to a plate in accordance with Test Example 5 using a microplate coated in Example 1 and a non-coating microplate.
Fig. 9 is a graph showing a result of measuring an insulin amount remained in a medium without adsorbing to a plate in accordance with Test Example 6 using a microplate coated in Example 1 and a non-coating microplate.
Fig. 10 is a graph showing a result of measuring a transferrin amount remained in a medium without adsorbing to a plate in accordance with Test Example 6 using a microplate coated in Example 1 and a non-coating microplate.
Fig. 11 is a graph showing a result of measuring a proliferation rate by culturing BM-CD34+ cells in the presence or absence of TPO (0, 0.01, 0.1, 1, 10 and 100 ng/mL) in accordance with Test Example 7 using a microplate coated in Example 3 and a non-coating microplate.
Fig. 12 is a graph showing an ED50 value of a TPO concentration to a cell proliferation rate in a microplate coated in Example 3 and a non-coating microplate calculated based on the result of Fig. 11.
Fig. 13 is a graph showing a result of measuring an IL6 amount remained in a medium without adsorbing to a plate in accordance with Test Example 8 using a microplate coated in Example 3 and a non-coating microplate.

### EMBODIMENTS TO CARRY OUT THE INVENTION

### «Explanation of the terms»

The terms used in the present invention have the following definitions, otherwise specifically mentioned.

In the present invention, the "halogen atom" means a fluorine atom, a chlorine atom, a bromine atom or an iodine atom.

In the present invention, the "alkyl group" means a linear or branched, saturated monovalent aliphatic hydrocarbon group. The "linear or branched alkyl group having 1 to 5 carbon atoms" may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group. The "linear or branched alkyl group having 1 to 18 carbon atoms" may be mentioned, in addition to the examples of the "linear or branched alkyl group having 1 to 5 carbon atoms", a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group or an octadecyl group, or an isomer thereof. Similarly, as the "linear or branched alkyl group having 1 to 10 carbon atoms" may be mentioned, in addition to the examples of the "linear or branched alkyl group having 1 to 5 carbon atoms", a hexyl group, a heptyl group, an octyl group, a nonyl group, a decyl group, or an isomer thereof.

In the present invention, the "linear or branched alkyl group having 1 to 5 carbon atoms which may be substituted by a halogen atom(s)" means either the above-mentioned linear or branched alkyl group having 1 to 5 carbon atoms, or the above-mentioned linear or branched alkyl group having 1 to 5 carbon atoms substituted by one or more of the above-mentioned halogen atoms. Examples of the "linear or branched alkyl group having 1 to 5 carbon atoms" are as mentioned above. On the other hand, the "linear or branched alkyl group having 1 to 5 carbon atoms substituted by one or more halogen atoms" means a group in which one or more optional hydrogen atoms of the above-mentioned linear or branched alkyl group having 1 to 5 carbon atoms is/are substituted by a halogen atom(s), and examples thereof may be mentioned a fluoromethyl group, a difluoromethyl group, a trifluoromethyl group, a chloromethyl group, a dichloromethyl group, a trichloromethyl group, a bromomethyl group, an iodomethyl group, a 2,2,2-trifluoroethyl group, a 2,2,2-trichloroethyl group, a perfluoroethyl group, a perfluorobutyl group or a perfluoropentyl group, etc.

In the present invention, the "ester bond" means -C(=O)-O- or -O-C(=O)-, the "amide bond" means -NHC(=O)- or -C(=O)NH- and the "ether bond" means -O-.

In the present invention, the "linear or branched alkylene group having 1 to 10 carbon atoms which may be substituted by a halogen atom(s)" means a linear or branched alkylene group having 1 to 10 carbon atoms or a linear or branched alkylene group having 1 to 10 carbon atoms substituted by one or more halogen atoms. Here, the "alkylene group" means a divalent organic group corresponding to the above-mentioned alkyl group. Examples of the "linear or branched alkylene group having 1 to 10 carbon atoms" may be mentioned a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, a hexamethylene group, an octamethylene group and a decamethylene group, etc., among these, an ethylene group, a propylene group, an octamethylene group and a decamethylene group are preferred, and, for example, a linear or branched alkylene group having 1 to 5 carbon atoms such as an ethylene group, a propylene group, a trimethylene group, a tetramethylene group, etc., are more preferred, and, in particular, an ethylene group or a propylene group is preferred. The "linear or branched alkylene group having 1 to 10 carbon atoms substituted by one or more halogen atoms" means a group in which one or more optional hydrogen atoms of the above-mentioned alkylene group is/are substituted by a halogen atom(s), and, in particular, a part or whole of the hydrogen atom(s) of the ethylene group or the propylene group is/are substituted by a halogen atom(s) is/are preferred.

In the present invention, the "alicyclic hydrocarbon group having 3 to 10 carbon atoms" means a monocyclic or polycyclic, saturated or partially unsaturated, monovalent aliphatic hydrocarbon group having 3 to 10 carbon atoms. Among these, a monocyclic or bicyclic, saturated monovalent aliphatic hydrocarbon group having 3 to 10 carbon atoms is preferred, and there may be mentioned, for example, a cycloalkyl group having 3 to 10 carbon atoms such as a cyclopropyl group, a cyclobutyl group and a cyclohexyl group, etc., or a bicycloalkyl group having 4 to 10 carbon atoms such as a bicyclo[3.2.1]octyl group, a bornyl group and an isobornyl group, etc.

In the present invention, the "aryl group having 6 to 10 carbon atoms" means a monocyclic or polycyclic, monovalent aromatic hydrocarbon group having 6 to 10 carbon atoms, and there may be mentioned, for example, a phenyl group, a naphthyl group or an anthryl group, etc. The "aryl group having 6 to 10 carbon atoms" may be substituted by one or more of the above-mentioned "linear or branched alkyl group having 1 to 5 carbon atoms which may be substituted by a halogen atom(s)."

In the present invention, the "aralkyl group having 7 to 15 carbon atoms" means a group -R-R' (where, R represents the above-mentioned "alkylene group having 1 to 5 carbon atoms", and R' represents the above-mentioned "aryl group having 6 to 10 carbon atoms"), and there may be mentioned, for example, a benzyl group, a phenethyl group or an α-methylbenzyl group, etc. The aryl portion of the "aralkyl group having 7 to 15 carbon atoms" may be substituted by one or more of the above-mentioned "linear or branched alkyl group having 1 to 5 carbon atoms which may be substituted by a halogen atom(s)."

In the present invention, the "aryloxyalkyl group having 7 to 15 carbon atoms" means a group -R-O-R' (where, R represents the above-mentioned "alkylene group having 1 to 5 carbon atoms", and R' represents the above-mentioned "aryl group having 6 to 10 carbon atoms"), and there may be mentioned, for example, a phenoxymethyl group, a phenoxyethyl group or a phenoxypropyl group, etc. The aryl portion of the "aryloxyalkyl group having 7 to 15 carbon atoms" may be substituted by one or more of the above-mentioned "linear or branched alkyl group having 1 to 5 carbon atoms which may be substituted by a halogen atom(s)."

In the present invention, the "halide ion" means a fluoride ion, a chloride ion, a bromide ion or an iodide ion.

In the present invention, the "inorganic acid ion" means a carbonate ion, a sulfate ion, a phosphate ion, a hydrogen phosphate ion, a dihydrogen phosphate ion, a nitrate ion, a perchlorate ion or a borate ion.

As the above-mentioned An⁻, preferred are a halide ion, a sulfate ion, a phosphate ion, a hydroxide ion and an isothiocyanate ion, and particularly preferred is a halide ion.

In the present invention, the (meth)acrylate compound means both of an acrylate compound and a methacrylate compound. For example, the (meth)acrylic acid means acrylic acid and methacrylic acid.

In addition, in the present invention, the "anion" or the "anionic group" means a negative ion or a negative ionic group, and also contains a group capable of becoming a negative ion or a negative ionic group by dissociating in water. Similarly, in the present invention, the "cation" or the "cationic group" means a positive ion or a positive ionic group, and also contains a group capable of becoming a positive ion or a positive ionic group by dissociating in water.

### <<Explanation of the present invention>>

The cell culture vessel of the present invention is not particularly limited as long as it is a material in which a coating containing a polymer having a peptide-adhesion inhibiting ability relating to cell proliferation is provided at least a part of the surface of the vessel.

### <Cell>

The cells in the present invention are not particularly limited as long as they are cells that perform proliferation peptide-dependently which relate to cell proliferation mentioned below. The cell is the most basic unit constituting an animal or plant, and has a cytoplasm and various kinds of cytoplasmic organelles inside a cell membrane as its elements. At this time, the nucleus including DNA may or may not be contained inside the cell. For example, in the cells derived from an animal in the present invention, germ cells such as sperm cells and egg cells, somatic cells constituting a living body, stem cells (multipotent stem cells, etc.), progenitor cells, cancer cells separated from a living body, cells (cell lines) separated from a living body and stably maintained outside the body by acquiring immortality, cells separated from a living body and artificially subjected to genetic modification, cells separated from a living body and the nucleus of which is artificially exchanged, etc., are contained. Examples of the somatic cells constituting a living body are not limited to the following, and include fibroblast, bone marrow cells, B lymphocytes, T lymphocytes, neutrophils, red blood cells, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, pancreatic beta cells, melanocytes, hematopoietic progenitor cells (for example, CD34-positive progenitor cells derived from umbilical cord blood or bone marrow), and mononuclear cells, etc. The somatic cells include, for example, cells collected from optional tissues such as skin, kidney, spleen, adrenal gland, liver, lung, ovary, pancreas, uterus, stomach, colon, small intestine, large intestine, bladder, prostate, testis, thymus, muscle, connective tissue, bone, cartilage, blood vessel tissue, blood (including umbilical cord blood), bone marrow, heart, eye, brain or nerve tissue, etc. The stem cell is a cell having both of an ability to replicate itself and an ability to differentiate into cells of other multiple lineages, and examples thereof include, while it is not limited to the following, embryonic stem cells (ES cell), embryonic tumor cells, embryonic germ stem cells, induced pluripotent stem cells (iPS cell), neural stem cells, hematopoietic stem cells, mesenchymal stem cells, hepatic stem cells, pancreatic stem cells, muscle stem cells, germ stem cells, intestinal stem cells, cancer stem cells, hair follicle stem cells, etc. As the multipotent stem cells, among the above-mentioned stem cells, ES cells, embryonic germ stem cells and iPS cells are mentioned. The progenitor cells are cells that are in the course of being differentiated from the above-mentioned stem cells into specific somatic cells or germ cells. The cancer cells are cells that have acquired unlimited proliferative capacity by deriving from somatic cells. The cell lines are cells that have acquired unlimited proliferative capacity by artificial manipulation *in vitro.* Examples of the cancer cell lines may be mentioned, while it is not limited to the following, HBC-4, BSY-1, BSY-2, MCF-7, MCF-7/ADR RES, HS578T, MDA-MB-231, MDA-MB-435, MDA-N, BT-549 and T47D as human breast cancer cell lines, HeLa as human cervical cancer cell lines, SKN as human uterine leiomyosarcoma cell lines, A549, EKVX, HOP-62, HOP-92, NCI-H23, NCI-H226, NCI-H322M, NCI-H460, NCI-H522, DMS273 and DMS114 as human lung cancer cell lines, Caco-2, COLO-205, HCC-2998, HCT-15, HCT-116, HT-29, KM-12, SW-620 and WiDr as human colon cancer cell lines, DU-145, PC-3 and LNCaP as human prostatic cancer cell lines, U251, SF-295, SF-539, SF-268, SNB-75, SNB-78 and SNB-19 as human central nervous system cancer cell lines, OVCAR-3, OVCAR-4, OVCAR-5, OVCAR-8, SK-OV-3 and IGROV-1 as human ovarian cancer cell lines, RXF-631L, ACHN, UO-31, SN-12C, A498, CAKI-1, RXF-393L, 786-0 and TK-10 as human renal cancer cell lines, MKN45, MKN28, St-4, MKN-1, MKN-7 and MKN-74 as human stomach cancer cell lines, LOX-IMVI, LOX, MALME-3M, SK-MEL-2, SK-MEL-5, SK-MEL-28, UACC-62, UACC-257 and M14 as skin cancer cell lines, and CCRF-CRM, K562, MOLT-4, HL-60TB, RPMI8226, SR, UT7/TPO, Jurkat and TF1 as leukemia cell lines, etc. Examples of the cell lines may be mentioned, while it is not limited to the following, HEK293 (human embryonic kidney cells), MDCK, MDBK, BHK, C-33A, AE-1, 3D9, Ns0/1, NIH3T3, PC12, S2, Sf9, Sf21, High Five (Registered Trademark) and Vero, etc. Examples of the human liver cell lines may be mentioned, while it is not limited to the following, HepG2, Hep3B, HepaRG (Registered Trademark), JHH7, HLF, HLE, PLC/PRF/5, WRL68, HB611, SK-HEP-1, HuH-4 and HuH-7, etc.

In the plant-derived cells, cells separated from each tissue of the plant body are included, and protoplasts obtained by artificially removing cell walls from the cells are also included.

### <Peptide relating to cell proliferation>

The peptide relating to cell proliferation in the present invention is not particularly limited as long as it is a peptide having a function of increasing a number, weight and/or volume, etc., of specific cells. The peptide means a material in which two or more amino acids are bonded by a peptide bond, and includes an oligopeptide having an amino acid number of 10 or less and a polypeptide having an amino acid number of 11 or more. As the peptide relating to cell proliferation according to the present invention, there may be mentioned a polypeptide preferably having an amino acid number of 200 or less, more preferably an amino acid number of 11 or more and 150 or less. Incidentally, the peptide may contain a non-amino acid portion, and may be linear or cyclic. The peptide relating to cell proliferation according to the present invention is preferably a peptide relating to cell proliferation through a receptor or a signal, or a peptide relating to nutrient transportation.

The peptide relating to cell proliferation through a receptor or a signal is preferably selected from the group consisting of a cell growth factor (also called as a growth factor), a hematopoietic factor, a neurotrophic factor, cytokines and hormones. Examples of the cell growth factor are not limited to the following, and may be mentioned an epidermal growth factor (EGF), an insulin-like growth factor (IGF), a transforming growth factor-β (TGF-β), a nerve growth factor (NGF), a brain-derived neurotrophic factor (BDNF), a vascular endothelial cell growth factor (VEGF), a hepatocyte growth factor (HGF), a platelet-derived growth factor (PDGF), an acidic fibroblast cell growth factor (aFGF), a basic fibroblast cell growth factor (bFGF), etc., and suitable examples may be mentioned bFGF, EGF and TGF-β used for culture of various kinds of stem cells such as human ES cells and iPS cells, etc. Examples of the a hematopoietic factor are not limited to the following, and may be mentioned a granulocyto colony-stimulating factor (G-CSF), a granulocyte-macrophage colony-stimulating factor (GM-CSF), a macrophage colony-stimulating factor (M-CSF), a monocyte chemotactic factor (MCP-1), a stem cell factor (SCF), a stem cell growth factor-a (SCGF-α), erythropoietin (EPO), thrombopoietin (TPO), etc. Examples of the neurotrophic factor may be mentioned a nerve growth factor (NGF), a brain-derived neurotrophic factor (BDNF), neurotrophin 3 and neurotrophin 4/5, etc. Examples of the cytokines may be mentioned interleukins, a colony stimulating factor, etc., and suitable examples may be mentioned interleukins. In particular, examples of the interleukin may be mentioned interleukin-la, interleukin-1β, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, a leukemia inhibitory factor (LIF), etc., and particularly suitable examples may be mentioned interleukin-3 and interleukin-6. Further, examples of the hormones are not limited to the following, and may be mentioned insulin, glucagon, adrenocorticotropic hormones, thyroid stimulating hormones, growth hormones, follicle stimulating hormones, luteotropic hormones, prolactin, fibronectin, etc.

Examples of the peptide relating to nutrient transportation are not limited to the following, and may be mentioned transferrin and bovine serum albumin (BSA).

In the present invention, "adhesion of the peptides relating to cell proliferation is inhibited" (or "having an ability to inhibit adhesion of the peptides relating to cell proliferation") means that, in the adhesion test carried out by the method described in Examples (for example, Test Example 6) mentioned below, a relative adhesion amount (%) ((the result of colorimetric determination of the culture medium of the coated vessel)/(the result of colorimetric determination of the culture medium of the non-coated vessel) × 100) when the result of colorimetric determination of the culture medium of the cell culture vessel to which a coating film is formed and the result of colorimetric determination of the culture medium of the cell culture vessel to which no coating film is formed are compared is 50% or less, preferably 30% or less, and further preferably 20% or less.

### <Coating>

In the present specification, the coating which inhibits adhesion of the peptides relating to cell proliferation contains a polymer such as a polymer of an ethylenically unsaturated monomer(s), or a polysaccharide or a derivative thereof. Examples of the polymer of the ethylenically unsaturated monomer may be mentioned one kind or two or more kinds of polymers of an ethylenically unsaturated monomer(s) selected from the group consisting of (meth)acrylic acid and an ester thereof; vinyl acetate; vinylpyrrolidone; ethylene; vinyl alcohol; and a hydrophilic functional derivative thereof. Examples of the polysaccharides or derivatives thereof may be mentioned a cellulose-based polymer such as hydroxyalkyl cellulose (for example, hydroxyethyl cellulose or hydroxypropyl cellulose), etc., starch, dextran and curdlan.

Examples of the hydrophilic functional group of the hydrophilic functional derivatives may be mentioned phosphoric acid, phosphonic acid and an ester structure thereof; a betaine structure; an amide structure; an alkylene glycol residue; an amino group; and a sulfinyl group, etc.

Here, phosphoric acid and an ester structure thereof mean the groups represented by the following formula: 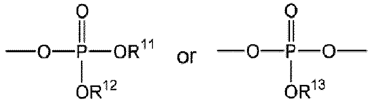 [where, R¹¹, R¹² and R¹³ each independently represent a hydrogen atom or an organic group (for example, a linear or branched alkyl group having 1 to 5 carbon atoms, etc.)],
and phosphonic acid and an ester structure thereof mean the groups represented by the following formula: 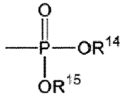 [where, R¹⁴ and R¹⁵ each independently represent a hydrogen atom or an organic group (for example, a linear or branched alkyl group having 1 to 5 carbon atoms, etc.)].
Examples of the ethylenically unsaturated monomer having such a structure may be mentioned acid phosphoxyethyl (meth)acrylate, vinylphosphonic acid, etc.

The betaine structure means a monovalent or a divalent group of a compound having an amphoteric center of a quaternary ammonium type cation structure and an acidic anion structure and may be mentioned, for example, a phosphorylcholine group: 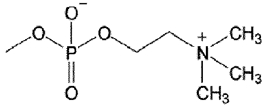 Examples of the ethylenically unsaturated monomer having such a structure may be mentioned 2-methacryloyloxyethylphosphorylcholine (MPC), etc.

The amide structure means a group represented by the following formula: 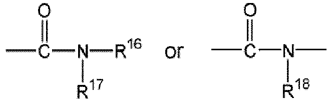 [where, R¹⁶, R¹⁷ and R¹⁸ each independently represent a hydrogen atom or an organic group (for example, a methyl group, hydroxymethyl group or hydroxyethyl group, etc.)]
Examples of the ethylenically unsaturated monomer having such a structure may be mentioned (meth)acrylamide, N-(hydroxymethyl) (meth)acrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; where, Alk is a linear or branched alkylene group having 1 to 10 carbon atoms) is/are subject to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc. Further, the monomer or the polymer having such a structure is disclosed in, for example, JP 2008-533489A, etc.

The amino group means a group represented by the formula: -NH₂, -NHR¹⁹ or -NR²⁰R²¹ [where, R¹⁹, R²⁰ and R²¹ each independently represent an organic group (for example, a linear or branched alkyl group having 1 to 5 carbon atoms, etc.)]. In the amino group of the present specification, a quaternized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 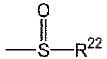 [where, R²² is an organic group (for example, an organic group having 1 to 10 carbon atoms, preferably an alkyl group having 1 to 10 carbon atoms which has one or more hydroxyl groups, etc.)].
As a polymer having such a structure, there may be mentioned a copolymer disclosed in the publication of JP 2014-48278A, etc.

Among these, as the polymer contained in the coating which inhibits adhesion of the peptides relating to cell proliferation, a copolymer containing a recurring unit which contains a group represented by the following formula (a) and a recurring unit which contains a group represented by the following formula (b): 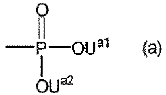 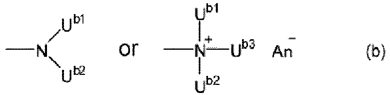 [wherein, U^{a1}, U^{a2}, U^{b1}, U^{b2} and U^{b3} each independently represent a hydrogen atom or a linear or branched alkyl group having 1 to 5 carbon atoms; An⁻ represents an anion selected from the group consisting of a halide ion, an inorganic acid ion, a hydroxide ion and an isothiocyanate ion] is preferable.

In addition, the copolymer may further contain a recurring unit which contains a group represented by the following formula (c):

-R^{c} (c)

[wherein R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (where, the above-mentioned aryl portion may be substituted by a linear or branched alkyl group having 1 to 5 carbon atoms which may be substituted by a halogen atom(s))].

The copolymer relating to the cell culture vessel of the present invention is not particularly limited as long as it is a copolymer containing a recurring unit which contains a group represented by the above-mentioned formula (a), a recurring unit which contains a group represented by the above-mentioned formula (b), and if necessary, a recurring unit which contains a group represented by the above-mentioned formula (c). Incidentally, in the present invention, the recurring unit which contains a group represented by the above-mentioned formula (c) is different from the recurring unit which contains a group represented by the above-mentioned formula (a) and the recurring unit which contains a group represented by the above-mentioned formula (b). The copolymer is desirably a material obtained by radical polymerization of a monomer which contains a group represented by the above-mentioned formula (a), a monomer which contains a group represented by the above-mentioned formula (b), and if necessary, a monomer which contains a group represented by the above-mentioned formula (c), but a material obtained by subjecting to polycondensation or polyaddition reaction may be also used. Examples of the copolymer may be mentioned a vinyl polymerized polymer in which olefins are reacted, polyamide, polyester, polycarbonate, polyurethane, etc., and among these, a vinyl polymerized polymer in which olefins are reacted or a (meth)acrylic polymer in which (meth)acrylate compounds are polymerized is desirable.

A ratio of the recurring unit which contains a group represented by the formula (a) in the copolymer is 3 mol% to 80 mol%. Incidentally, the copolymer may contain two or more kinds of the recurring units which contain a group represented by the formula (a).

A ratio of the recurring unit which contains a group represented by the formula (b) in the copolymer is 3 mol% to 80 mol%. Incidentally, the copolymer may contain two or more kinds of the recurring units which contain a group represented by the formula (b).

A ratio of the recurring unit which contains a group represented by the formula (c) in the copolymer may be the reminder subtracting the ratio of the above-mentioned formulae (a) and (b) based on the whole copolymer and is, for example, 0 mol% to 90 mol%. Incidentally, the copolymer may contain two or more kinds of the recurring units which contain a group represented by the formula (c).

A preferred embodiment of the copolymer relating to the cell culture vessel of the present invention is a copolymer containing the recurring units represented by the following formulae (a1) and (b1). 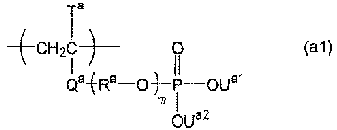 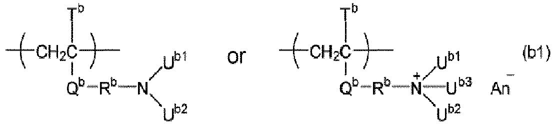 wherein, T^{a} and T^{b} each independently represent a hydrogen atom or a linear or branched alkyl group having 1 to 5 carbon atoms, Q^{a} and Q^{b} each independently represent a single bond, an ester bond or an amide bond, R^{a} and R^{b} each independently represents a linear or branched alkylene group having 1 to 10 carbon atoms which may be substituted by a halogen atom(s), U^{a1}, U^{a2}, U^{b1}, U^{b2} and U^{b3} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion, and m represents an integer of 0 to 6.

The copolymer may further contain a recurring unit which contains a group represented by the following formula (c1).

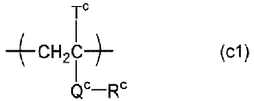

In the formula, T^{c} represents a hydrogen atom or a linear or branched alkyl group having 1 to 5 carbon atoms, Q^{c} represents a single bond, an ether bond or an ester bond, R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (where, the above-mentioned aryl portion may be substituted by a linear or branched alkyl group having 1 to 5 carbon atoms which may be substituted by a halogen atom(s)).

In the formula (a1), m represents an integer of 0 to 6, preferably represents an integer of 1 to 6, more preferably represents an integer of 1 to 5, and particularly preferably 1.

A ratio of the recurring unit is represented by the formula (a1) in the copolymer is 3 mol% to 80 mol%. Incidentally, the copolymer may contain two or more kinds of the recurring units represented by the formula (a1).

A ratio of the recurring unit is represented by the formula (b1) in the copolymer is 3 mol% to 80 mol%. Incidentally, the copolymer may contain two or more kinds of the recurring units represented by the formula (b1).

A ratio of the recurring unit is represented by the formula (c1) contained in the copolymer may be the reminder subtracting the ratio of the above-mentioned formula (a1) and the formula (b1) based on the whole copolymer and is, for example, 0 mol% to 90 mol%. Incidentally, the copolymer may contain two or more kinds of the recurring units represented by the formula (c1).

A preferred another embodiment of the copolymer relating to the cell culture vessel of the present invention is a copolymer which is obtained by reacting (polymerizing) a monomer mixture containing compounds represented by the following formulae (A) and (B): 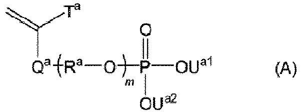 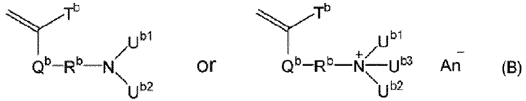 [wherein,
T^{a} and T^{b} each independently represent a hydrogen atom or a linear or branched alkyl group having 1 to 5 carbon atoms;
Q^{a} and Q^{b} each independently represent a single bond, an ester bond or an amide bond;
R^{a} and R^{b} each independently represent a linear or branched alkylene group having 1 to 10 carbon atoms which may be substituted by a halogen atom(s);
U^{a1}, U^{a2}, U^{b1}, U^{b2} and U^{b3} each independently represent a hydrogen atom or a linear or branched alkyl group having 1 to 5 carbon atoms;
An⁻ represents an anion selected from the group consisting of a halide ion, an inorganic acid ion, a hydroxide ion and an isothiocyanate ion; and
m represents an integer of 0 to 6]
in a solvent.

The copolymer may be a copolymer obtained from a monomer mixture which further contains a compound represented by the following formula (C): 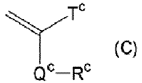 [wherein,
T^{c} represents a hydrogen atom or a linear or branched alkyl group having 1 to 5 carbon atoms;
Q^{c} represents a single bond, an ether bond or an ester bond; and
R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms (where, the above-mentioned aryl portion may be substituted by a linear or branched alkyl group having 1 to 5 carbon atoms which may be substituted by a halogen atom(s))].

T^{a}, T^{b} and T^{c} are each preferably a hydrogen atom, a methyl group or an ethyl group, and more preferably a hydrogen atom or a methyl group. Q^{a}, Q^{b} and Q^{c} are each preferably a single bond or an ester bond, and more preferably an ester bond. R^{a} and R^{b} are each preferably a linear or branched alkylene group having 1 to 5 carbon atoms, and more preferably a methylene group, an ethylene group or a propylene group. R^{c} is preferably a linear or branched alkyl group having 4 to 18 carbon atoms or a cycloalkyl group having 3 to 10 carbon atoms, and more preferably a butyl group, a pentyl group, a hexyl group or an isomer thereof, or a cyclohexyl group. U^{a1}, U^{a2}, U^{b1}, U^{b2} and U^{b3} are each preferably a hydrogen atom, a methyl group, an ethyl group or a t-butyl group, U^{a1} and U^{a2} of the formula (a) is more preferably a hydrogen atom, and U^{b1}, U^{b2} and U^{b3} of the formula (b) is more preferably a hydrogen atom, a methyl group, an ethyl group or a t-butyl group.

Specific examples of the above-mentioned formula (A) may be mentioned vinylphosphonic acid, acid phosphoxyethyl (meth)acrylate, 3-chloro-2-acid phosphoxypropyl (meth)acrylate, acid phosphoxypropyl (meth)acrylate, acid phosphoxymethyl (meth)acrylate, acid phosphoxypolyoxyethylene glycol mono(meth)acrylate, acid phosphoxypolyoxypropylene glycol mono(meth)acrylate, etc., among these, vinylphosphonic acid, acid phosphoxyethyl methacrylate (=2-(methacryloyloxy)ethyl phosphate) or acid phosphoxypolyoxyethylene glycol monomethacrylate are preferably used, and most preferably acid phosphoxyethyl methacrylate (=2-(methacryloyloxy)ethyl phosphate).

The structural formulae of vinylphosphonic acid, acid phosphoxyethyl methacrylate (=2-(methacryloyloxy)ethyl phosphate), acid phosphoxypolyoxyethylene glycol monomethacrylate and acid phosphoxypolyoxypropylene glycol monomethacrylate are represented by the following formula (A-1) to the formula (A-4), respectively.

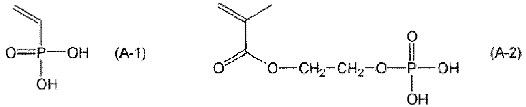

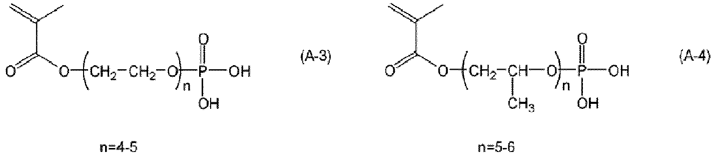

These compounds may contain a (meth)acrylate compound having two functional groups as represented by the general formula (D) or (E) mentioned later at the time of synthesis in some cases.

Specific examples of the above-mentioned formula (B) may be mentioned dimethylaminoethyl (meth)acrylate, diethylaminoethyl(meth)acrylate, dimethylaminopropyl(meth)acrylate, 2-(t-butylamino)ethyl(meth)acrylate, methacryloylcholine chloride, etc., among these, dimethylaminoethyl(meth)acrylate, methacryloylcholine chloride or 2-(t-butylamino)ethyl(meth)acrylate is preferably used, and dimethylaminoethyl(meth)acrylate is most preferably used.

The structural formulae of dimethylaminoethyl acrylate (=acrylic acid 2-(dimethylamino)ethyl), diethylaminoethyl methacrylate (=methacrylic acid 2-(diethylamino)ethyl), dimethylaminoethyl methacrylate (=methacrylic acid 2-(dimethylamino)ethyl), methacryloylcholine chloride and 2-(t-butylamino)ethyl methacrylate (=methacrylic acid 2-(t-butylamino)ethyl) are represented by the following formula (B-1) to the formula (B-5), respectively.

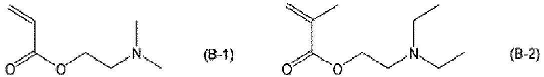

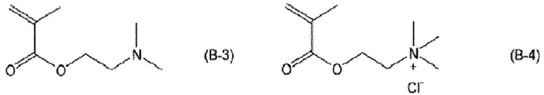

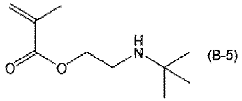

Specific examples of the above-mentioned formula (C) may be mentioned linear or branched alkyl esters of (meth)acrylic acid such as butyl (meth)acrylate, 2-ethylhexyl (meth)acrylate, lauryl (meth)acrylate, stearyl (meth)acrylate, etc.; cyclic alkyl esters of (meth)acrylic acid such as cyclohexyl (meth)acrylate, isobornyl (meth)acrylate, etc.; aralkyl esters of (meth)acrylic acid such as benzyl (meth)acrylate, phenethyl (meth)acrylate, etc.; styrene-based monomers such as styrene, methylstyrene, chloromethylstyrene, etc.; vinyl ether-based monomers such as methyl vinyl ether, butyl vinyl ether, etc.; and vinyl ester-based monomers such as vinyl acetate, vinyl propionate, etc. Among these, butyl (meth)acrylate or cyclohexyl (meth)acrylate is preferably used.

The structural formulae of butyl methacrylate (=methacrylic acid butyl) and cyclohexyl methacrylate (=methacrylic acid cyclohexyl) are represented by the following formula (C-1) and the formula (C-2), respectively.

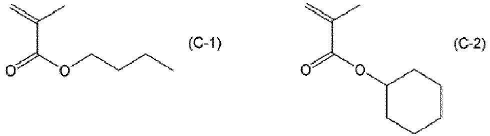

In another embodiment of the copolymer according to the present invention, in addition to the compounds represented by the above-mentioned formulae (A), (B) and, if necessary, (C), an optional fourth component may be further copolymerized. For example, as the fourth component, a (meth)acrylate compound having two or more functional groups may be copolymerized, and a part of the polymer may be partially three-dimensionally crosslinked. Such a fourth component may be mentioned, for example, a bifunctional monomer represented by the following formula (D) or (E): 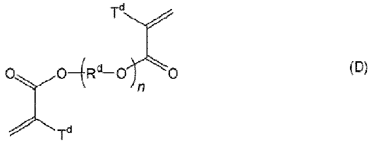 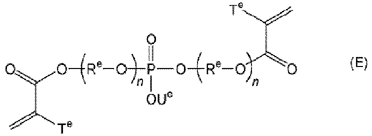 [wherein, T^{d}, T^{e} and U^{e} each independently represent a hydrogen atom or a linear or branched alkyl group having 1 to 5 carbon atoms, R^{d} and R^{e} each independently represent a linear or branched alkylene group having 1 to 10 carbon atoms which may be substituted by a halogen atom(s); and n represents an integer of 1 to 6]. That is, the copolymer relating to the present invention preferably contains a crosslinking structure derived from such a bi-functional monomer.

In the formulae (D) and (E), T^{d} and T^{e} are preferably each independently a hydrogen atom, a methyl group or an ethyl group, and more preferably each independently a hydrogen atom or a methyl group.

In the formula (E), U^{e} is preferably a hydrogen atom, a methyl group or an ethyl group, and more preferably a hydrogen atom.

In the formula (D), R^{d} preferably represents a linear or branched alkylene group having 1 to 3 carbon atoms which may be substituted by a halogen atom(s), more preferably each independently represent an ethylene group or a propylene group, or an ethylene group or a propylene group substituted by one chlorine atom, and particularly preferably an ethylene group or a propylene group. In addition, in the formula (D), n preferably represents an integer of 1 to 5, and particularly preferably 1.

In the formula (E), R^{e} preferably represents a linear or branched alkylene group having 1 to 3 carbon atoms which may be substituted by a halogen atom(s), more preferably each independently represent an ethylene group or a propylene group, or an ethylene group or a propylene group substituted by one chlorine atom, and particularly preferably an ethylene group or a propylene group. Also, in the formula (E), n preferably represents an integer of 1 to 5, and particularly preferably 1.

The bifunctional monomer represented by the formula (D) may be preferably mentioned ethylene glycol di(meth)acrylate, triethylene glycol di(meth)acrylate, and propylene glycol di(meth)acrylate.

The bifunctional monomer represented by the formula (E) may be preferably mentioned bis(methacryloyloxymethyl) phosphate, bis[(2-methacryloyloxy)ethyl] phosphate, bis[3-(methacryloyloxy)propyl] phosphate, or a bifunctional monomer derived from the above-mentioned formula (A-3) or (A-4).

Among the formula (D) and the formula (E), the bifunctional monomer represented by the formula (E) is more preferably used.

In addition, as the trifunctional (meth)acrylate compound, there may be mentioned phosphinylidynetris(oxy-2,1-ethanediyl) triacrylate.

Among these fourth components, in particular, among ethylene glycol dimethacrylate, and the bi-functional monomer derived from the above-mentioned formulae (A-3) and (A-4), a dimethacrylate having a recurring unit of ethylene glycol and propylene glycol, and among bis[2-(methacryloyloxy)ethyl] phosphate and the bi-functional monomer derived from the above-mentioned formulae (A-3) and (A-4), a dimethacrylate having a recurring unit of ethylene glycol and propylene glycol through a phosphate ester group are preferable, and the structural formulae thereof are represented by the following formulae (D-1) to (D-3) and formulae (E-1) to (E-3), respectively.

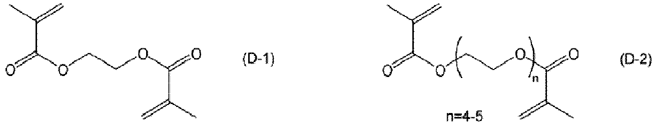

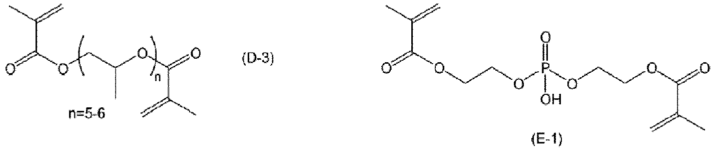

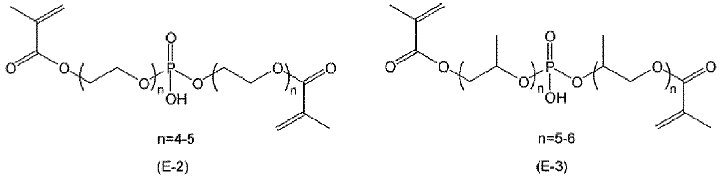

Among these, the formulae (E-1) to (E-3) are particularly preferable.

In the copolymer, one or two or more kinds of these fourth components may be contained.

A ratio of the fourth component in the above-mentioned copolymer, for example, a crosslinking structure derived from the bi-functional monomer represented by the above-mentioned formula (D) or (E) is 0 mol% to 50 mol%, preferably5 mol% to 45 mol%, and most preferably 10 mol% to 40 mol%.

A ratio of the compound represented by the formula (A) in the whole monomer constituting the above-mentioned copolymer is 3 mol% to 80 mol%. In addition, the compound represented by the formula (A) may be two or more kinds.

A ratio of the compound represented by the formula (B) in the whole monomer constituting the above-mentioned copolymer is 3 mol% to 80 mol%. In addition, the compound represented by the formula (B) may be two or more kinds.

A ratio of the compound represented by the formula (C) in the whole monomer constituting the above-mentioned copolymer may be the reminder subtracting the ratio of the above-mentioned formulae (A) and (B) and is, for example, 0 mol% to 90 mol%. In addition, the compound represented by the formula (C) may be two or more kinds.

The copolymer according to the embodiments of the present invention can be synthesized by the methods of radical polymerization, anion polymerization, cation polymerization, etc., which are general synthetic methods of an acrylic polymer or a methacrylic polymer, etc. The form can be taken various kinds of methods such as solution polymerization, suspension polymerization, emulsion polymerization, bulk polymerization, etc.

As the solvent for the polymerization reaction, it may be water, phosphate-buffered saline or an alcohol such as ethanol, etc., or a mixed solvent in which these are combined, and desirably contains water or ethanol. Further, it is preferable to contain 10% by mass or more and 100% by mass or less of water or ethanol. Moreover, it is preferable to contain 50% by mass or more and 100% by mass or less of water or ethanol. Furthermore, it is preferable to contain 80% by mass or more and 100% by mass or less of water or ethanol. Still further, it is preferable to contain 90% by mass or more and 100% by mass or less of water or ethanol. It is preferable that the sum of water and ethanol is 100% by mass.

As a reaction concentration, for example, a concentration of the compound represented by the above-mentioned formula (A) or the formula (B) in the reaction solvent is preferably made 0.01% by mass to 4% by mass. If the concentration exceeds 4% by mass, for example, the copolymer sometimes gelled in the reaction solvent due to strong association property possessed by the phosphate group represented by the formula (A). If the concentration is less than 0.01% by mass, a concentration of the obtained varnish is too low, so that it is difficult to prepare a coating agent for obtaining a coating film with a sufficient film thickness. The concentration is more preferably 0.01% by mass to 3% by mass, for example, 3% by mass, 2% by mass or 1% by mass.

Also, in the synthesis of the copolymer according to the present invention, for example, after making a salt described in the following formula (1), polymerization is carried out with the compound represented by the formula (C), in some cases, to prepare a copolymer.

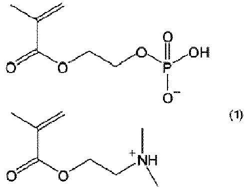

The phosphate group-containing monomer is a monomer easily associated, so that when it is to be added dropwise in the reaction system, it may be added dropwise in the reaction solvent little by little so as to rapidly disperse therein.

Further, the reaction solvent may be heated (for example, 40°C to 100°C) so as to raise solubility of the monomer and the polymer.

In order to efficiently proceed the polymerization reaction, it is desirable to use a polymerization initiator. Examples of the polymerization initiator may be used 2,2'-azobis(isobutyronitrile), 2,2'-azobis(2-methylbutyronitrile), 2,2'-azobis(2,4-dimethylvaleronitrile) (available from Wako Pure Chemical Industries, Ltd.; VA-065, 10 hour half-life temperature; 51°C), 4,4'-azobis(4-cyanovaleric acid), 2,2'-azobis(4-methoxy-2,4-dimethylvaleronitrile), 1,1'-azobis(cyclohexane-l-carbonitrile), 1-[(1-cyano-1-methylethyl)azo]formamide, 2,2'-azobis[2-(2-imidazolin-2-yl)propane], 2,2'-azobis(2-methylpropionamidine) dihydrochoride, 2,2' -azobis[2-methyl-N-(2-hydroxyethyl)propionamide] (available from Wako Pure Chemical Industries, Ltd.; VA-086, 10 hour half-life temperature; 86°C), benzoyl peroxide (BPO), 2,2'-azobis[N-(2-carboxyethyl)-2-methylpropionamidine] n-hydrate (available from Wako Pure Chemical Industries, Ltd.; VA-057, 10 hour half-life temperature; 57°C), 4,4'-azobis(4-cyanopentanoic acid) (available from Wako Pure Chemical Industries, Ltd.; V-501), 2,2'-azobis[2-(2-imidazolin-2-yl)propane] dihydrochloride (available from Wako Pure Chemical Industries, Ltd.; VA-044, 10 hour half-life temperature; 44°C), 2,2'-azobis[2-(2-imidazolin-2-yl)propane] disulfate dihydrate (available from Wako Pure Chemical Industries, Ltd.; VA-046B, 10 hour half-life temperature; 46°C), 2,2'-azobis[2-(2-imidazolin-2-yl)propane] (available from Wako Pure Chemical Industries, Ltd.; VA-061, 10 hour half-life temperature; 61°C), 2,2'-azobis(2-amidinopropane) dihydrochloride (available from Wako Pure Chemical Industries, Ltd.; V-50, 10 hour half-life temperature; 56°C), peroxydisulfuric acid or t-butylhydroperoxide, etc.

When solubility in water, ion balance and interaction with the monomer are taking into consideration, it is preferably selected from 2,2'-azobis[2-methyl-N-(2-hydroxyethyl)propionamide], 2,2'-azobis[N-(2-carboxyethyl)-2-methylpropionamidine] n-hydrate, 4,4'-azobis(4-cyanopentanoic acid), 2,2'-azobis[2-(2-imidazolin-2-yl)propane] dihydrochloride, 2,2'-azobis[2-(2-imidazolin-2-yl)propane] disulfate dihydrate, 2,2'-azobis[2-(2-imidazolin-2-yl)propane], 2,2'-azobis(2-amidinopropane) dihydrochloride and peroxydisulfuric acid.

When solubility in an organic solvent, ion balance and interaction with the monomer are taking into consideration, it is desirable to use 2,2'-azobis(2,4-dimethylvaleronitrile) or 2,2'-azobis(isobutyronitrile).

As an amount of the polymerization initiator to be added, it is 0.05% by mass to 10% by mass based on the total weight of the monomer.

The reaction conditions are to carry out stirring in a reaction container by heating to 50°C to 200°C with an oil bath, etc., for 1 hour to 48 hours, more preferably at 80°C to 150°C for 5 hours to 30 hours to proceed the polymerization reaction whereby a copolymer according to the present invention can be obtained. The reaction atmosphere is preferably a nitrogen atmosphere.

As a reaction procedure, whole reaction substances may be charged in the reaction solvent at room temperature and then these may be heated to the above-mentioned temperature and polymerized, or all or a part of the mixture of the reaction substances may be added dropwise little by little into a previously heated solvent.

According to the latter reaction procedure, the copolymer of the present invention can be prepared by the producing method including the steps of adding a mixture containing the compounds of the above-mentioned formulae (A), (B) and, if necessary, (C), the solvent and the polymerization initiator dropwise into a solvent maintained at a temperature higher than the 10 hour half-life temperature of the polymerization initiator, and reacting (polymerizing) the same.

The molecular weight of the copolymer according to the present invention may be about several thousands to several millions, and preferably 5,000 to 5,000,000. It is further preferably 10,000 to 2,000,000, and most preferably 5,000 to 1,000,000. In addition, it may be either of a random copolymer, a block copolymer or a graft copolymer, there is no particular limitation on the copolymerization reaction itself for manufacturing the copolymer, and a conventionally known method synthesizing in a liquid can be used such as polymerization utilizing radical polymerization, ionic polymerization, photopolymerization, emulsion polymerization, etc. These can be used any of the copolymers of the present invention solely or a plurality of the copolymers are mixed and the ratio thereof may be changed depending on the objective uses.

The coating agent to be used for forming a coating onto the cell culture vessel according to the present invention may be prepared by diluting a desired copolymer with a desired solvent to a predetermined concentration, depending on the cases.

Such a solvent may be mentioned water, phosphate-buffered saline (PBS) and an alcohol. The alcohol may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, which may be used alone or a mixed solvent of a combination thereof, and from the viewpoint of dissolution of the copolymer, it is preferably selected from water, PBS, ethanol, propanol and a mixed solvent thereof, and more preferably selected from water, ethanol and a mixed solvent thereof.

Further, the coating agent may be prepared from the copolymer-containing varnish. The copolymer-containing varnish can be prepared, for example, by a manufacturing method which contains a step of reacting (polymerizing) the compounds represented by the above-mentioned formulae (A), (B) and, if necessary, (C), in a solvent, with a total concentration of the compounds of 0.01% by mass to 20% by mass.

A concentration of the solid content in the coating agent is desirably 0.01 to 50% by mass to uniformly form a coating film. Also, a concentration of the copolymer in the coating agent is preferably 0.01 to 4% by mass, more preferably 0.01 to 3% by mass, particularly preferably 0.01 to 2% by mass, and further preferably 0.01 to 1% by mass. If the concentration of the copolymer is less than 0.01% by mass, a coating film which has a sufficient film thickness cannot be formed since the concentration of the copolymer in the coating agent is too low, while if it exceeds 4% by mass, storage stability of the coating agent becomes worse, and there is a possibility to cause precipitation or gellation of the dissolved material.

Incidentally, to the coating agent, in addition to the above-mentioned copolymer and the solvent, other substances may be added within the range that does not impair the properties of the obtainable coating, if necessary. As the other substances, there may be mentioned preservatives, surfactants, primers that enhance adhesive property with the substrate material, fungicides and sugars, etc.

In order to adjust ion balance of the copolymer in the coating agent, a step of adjusting a pH in the coating agent in advance may be contained. Adjustment of the pH may be carried out, for example, by adding a pH adjusting agent to the composition containing the above-mentioned copolymer and the solvent and made the pH of the composition 3.0 or more, preferably 3.5 to 8.5, and further preferably 3.5 to 8.0. A kind of the usable pH adjusting agent and an amount thereof can be optionally selected depending on the concentration of the above-mentioned copolymer or an existing ratio of the anion and the cation thereof, and the like.

Examples of the pH adjusting agent may be mentioned organic amines such as ammonia, diethanolamine, pyridine, N-methyl-D-glucamine, tris(hydroxymethyl)-aminomethane, etc.; alkali metal hydroxides such as potassium hydroxide, sodium hydroxide, etc.; alkali metal halides such as potassium chloride, sodium chloride, etc.; inorganic acids such as sulfuric acid, phosphoric acid, hydrochloric acid, carbonic acid, etc., or alkali metal salts thereof; quaternary ammonium cations such as choline, etc.; or a mixture thereof (for example, a buffer such as phosphate buffered saline, etc.). Among these, ammonia, diethanolamine, sodium hydroxide, choline, N-methyl-D-glucamine, tris(hydroxymethyl)aminomethane are preferable, and in particular, ammonia, diethanolamine, sodium hydroxide and choline are preferable.

The cell culture vessel of the present invention has a coating formed by the above-mentioned coating agent at least a part of the surface of the cell culture vessel. Specifically, it has the coating at least a part of the surface at the inside and/or outside of the vessel, which can be contacted with a culture medium containing cells and peptides relating to cell proliferation.

### <Vessel>

The vessel to which the copolymer is coated, which constitutes the cell culture vessel of the present invention, may be vessels with an optional shape capable of using in this field of the art, and may be mentioned, for example, dishes or schale generally used for cell culture such as petri dishes, dishes for tissue culture, multi dishes, etc., flasks such as a cell culture flask, a spinner flask, etc., bags such as plastic bags, Teflon (Registered Trademark) bags, culture bags, etc., plates such as microplates, microwell plates, multi plates, multiwell plates, etc., and bottles such as chamber slide, tubes, trays, roller bottles and the like. It is preferably mentioned 6 to 1536 well multi-well plates and schale.

Also, the material of the vessel may be used glass, a metal, a metal containing compound or a semi-metal containing compound, activated charcoal or a resin. The metal may be mentioned a typical metal: (an alkali metal: Li, Na, K, Rb, Cs; an alkaline earth metal: Ca, Sr, Ba, Ra), a magnesium group element: Be, Mg, Zn, Cd, Hg; an aluminum group element: Al, Ga, In; a rare earth element: Y, La, Ce, Pr, Nd, Sm, Eu; a tin group element: Ti, Zr, Sn, Hf, Pb, Th; an iron group element: Fe, Co, Ni; a vanadium group element: V, Nb, Ta, a chromium group element: Cr, Mo, W, U; a manganese group element: Mn, Re; a noble metal: Cu, Ag, Au; and a platinum group element: Ru, Rh, Pd, Os, Ir, Pt, etc. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned, for example, cellulose, cellulose triacetate (CTA), nitrocellulose (NC), cellulose to which dextran sulfate has been fixed, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra high molecular weight polyethylene (UHPE), polydimethylsiloxane (PDMS), acrylonitrile-butadiene-styrene resin (ABS) or Teflon (Registered Trademark). In the manufacture of the cell culture vessel of the present invention, at the time of coating the copolymer to exist at least a part of the surface of the vessel, it is not necessary to treat it at a high temperature, so that a resin having low heat resistance, etc., can be also applied.

A material(s) of the vessel may be one kind or a combination of two or more kinds. Among these materials, it is preferably glass, silicon, silicon oxide, polystyrene (PS), polypropylene (PP), polyether sulfone (PES), polyethylene terephthalate (PET), polycarbonate (PC), polyvinyl chloride (PVC), Teflon (Registered Trademark), cycloolefin polymer (COP), polydimethylsiloxane (PDMS) or stainless (SUS304, SUS316, SUS316L, etc.) alone, or a combination selected from these, and particularly preferably glass, polystyrene (PS), polypropylene (PP) or stainless (SUS304, SUS316, SUS316L, etc.).

### <Manufacturing method of cell culture vessel>

The present invention relates to a method for manufacturing a cell culture vessel having a coating onto at least a part of the surface of the vessel, which comprises a step of contacting the coating agent as mentioned above with at least a part of the surface of the vessel. There is no particular limitation on the contact of the coating agent and the surface of the vessel, and there may be used a method in which the vessel is dipped in the coating agent, the coating agent is added to the vessel and allowed to stand for a predetermined time, or coating the coating agent onto the surface of the vessel, etc., and a method of adding the coating agent to the vessel and allowing to stand for a predetermined time is preferable. Addition can be carried out, for example, by adding the coating agent with 0.5 to 1-fold amount of the whole volume of the vessel using a syringe, etc. Standing is carried out by appropriately selecting a time and a temperature depending on the material of the vessel and the kind of the coating agent and carried out, for example, for 5 minutes to 24 hours, preferably for 30 minutes to 3 hours, at 10 to 35°C, preferably at 20 to 30°C, and most preferably at 25°C. According to the procedure, a cell culture vessel having a coating onto at least a part of the surface of the vessel, preferably whole surface thereof can be manufactured.

Also, the coating on the surface of the vessel obtained by such a method can be used as a cell culture vessel after the step of contacting with at least a part of the above-mentioned surface of the vessel, preferably after the step of adding a coating agent and allowing to stand for a predetermined time, without passing through the drying step as such, or after the step of washing using water or a medium (for example, water, buffer, medium, etc.) of a sample to be applied to cell culture.

That is, after the step of contacting at least a part of the above-mentioned surface of the vessel, preferably after the step of adding a coating agent and allowing to stand for a predetermined time, within 48 hours, preferably within 24 hours, further preferably within 12 hours, further preferably within 6 hours, further preferably within 3 hours, further preferably within 1 hour, without passing through the drying step as such, or after washing using water or a medium (for example, water, buffer, medium, etc., particularly preferably medium (for example, DMEM medium (Dulbecco's modified Eagle medium)) of a sample to be applied to cell culture, it can be used as a cell culture vessel.

The vessel may be applied to a drying step. The drying step is carried out under atmosphere or under vacuum, preferably at a temperature in the range of -200°C to 200°C. By the drying step, the solvent in the above-mentioned coating agent is removed, and also the formula (a) and the formula (b) of the copolymer relating to the present invention are formed an ion bonding with each other to completely adhere to the substrate.

The coating can be formed, for example, by the drying at room temperature (10°C to 35°C, preferably at 20°C to 30°C, for example, 25°C), and it may be dried, for example, at 40°C to 50°C to form the coating more rapidly. In addition, a drying step by the freeze-drying method at an extremely low temperature to a low temperature (-200°C to around -30°C) may be used. The freeze drying is called as vacuum freeze-drying, and is a method in which a material to be desired to dry is cooled with a refrigerant in general and remove the solvent under a vacuum state by sublimation. A general refrigerant to be used in the freeze drying may be mentioned a mixed medium of dry ice and methanol (-78°C), liquid nitrogen (-196°C), etc.

If the drying temperature is lower than -200°C, an unusual refrigerant must be used which lacks versatility, and a long time is required for solvent sublimation so that efficiency is poor. If the drying temperature exceeds 200°C, ionic bonding reaction on the surface of the coating proceeds excessively, whereby the surface loses hydrophilicity and adhesion inhibiting ability of the cells and the peptides relating to cell proliferation is not exhibited. More preferred drying temperature is 10°C to 180°C, and more preferred drying temperature is 25°C to 150°C.

The coating of the present application is produced through the simple and easy step as mentioned above.

In addition, in order to remove impurities, unreacted monomer, etc., remained in the coating, and further, to adjust ion balance of the copolymer in the coating, it may be carried out a step of washing with at least one kind of the solvent selected from water and an aqueous solution containing an electrolyte. Washing is desirably running water washing or ultrasonic wave washing, etc. The above-mentioned water and the aqueous solution containing an electrolyte may be a material heated, for example, in the range of 40°C to 95°C. The aqueous solution containing an electrolyte is preferably PBS, physiological saline (containing only sodium chloride), Dulbecco's phosphate-buffered saline, Tris buffered physiological saline, HEPES buffered physiological saline and veronal buffered physiological saline, and particularly preferably PBS. After adhesion, the coating film does not elute even when washed with water, PBS and alcohol, etc., and still remains firmly adhered to the substrate. Even if cells or proteins are adhered to the formed coating, these can be easily removed by washing, etc., thereafter, so that the surface of the vessel onto which the coating of the present invention has been formed has an ability to inhibit adhesion of cells and peptides relating to cell proliferation.

A film thickness of the coating applied to the surface of the vessel of the present invention can be appropriately adjusted according to the shape of the vessel or the kind of the sample, etc., and may be substantially uniform over the whole surface of the vessel or may be partially ununiform, which is not particularly limited, and preferably 10 to 1000Å, further preferably 10 to 500Å, and most preferably 10 to 300Å.

### <Other embodiments>

The present invention also relates to a method for increasing a proliferation rate of cells cultured in a cell culture vessel by subjecting a coating which inhibits adhesion of a peptide relating to cell proliferation to at least a part of the surface of the vessel. Increasing a proliferation rate of cells means, for example, in a cell proliferation test carried out in accordance with the method described in Test Example 1 of Examples, the proliferation rate of the cells is increased 5% or more, preferably 10% or more, and further preferably 50% or more as compared with the control (non-coating). By applying a coating which inhibits adhesion of the peptide relating to cell proliferation onto at least a part of the surface of the cell culture vessel, adhesion of the peptide relating to cell proliferation is inhibited, and during the culture period, the peptide relating to cell proliferation can exist sufficiently in the culture medium, so that the proliferation rate of cells to be cultured in the vessel is significantly increased. The meanings of cells, peptides relating to cell proliferation, coatings and vessels, etc., in such a method are as defined above.

The present invention also relates to a method for reducing an amount of a peptide relating to cell proliferation into a cell culture vessel by subjecting a coating which inhibits adhesion of a peptide relating to cell proliferation to at least a part of the surface of the vessel. Reducing an amount of a peptide means that, for example, in a method in accordance with Test Example 1 of Examples, Effective dose 50 (ED50) value of the peptide concentration based on the cell proliferation rate is 50% or less, and preferably 40% or less as compared with the control (non-coating). By applying a coating which inhibits adhesion of the peptide relating to cell proliferation onto at least a part of the surface of the cell culture vessel, adhesion of the peptide relating to cell proliferation is inhibited, so that it is not necessary to consider an adhered amount at the time of determining the added amount, whereby the added amount of the peptide relating to cell proliferation can be reduced. The meanings of cells, peptides relating to cell proliferation, coatings and vessels, etc., in such a method are as defined above.

### EXAMPLES

In the following, the present invention is explained in more detail based on Synthetic Examples, Examples, Test Examples, etc., but the present invention is not limited by these.

### <Synthetic Example 1>

While cooling 25.00 g of acid phosphoxyethyl methacrylate (product name; Phosmer M, available from UniChemical Co., non-volatile content by evaporation to dryness method at 100°C for 1 hour: 91.8%, a mixture of acid phosphoxyethyl methacrylate (44.2% by mass), bis[2-(methacryloyloxy)ethyl] phosphate (28.6% by mass) and other substance(s) (27.2% by mass)) at 35°C or lower, 29.95 g of choline (48-50% aqueous solution: available from Tokyo Chemical Industry Co., Ltd.) was added thereto and the mixture was stirred until the mixture became uniform. To the mixed solution were successively added 20.95 g of 80% aqueous methacroylcholine chloride solution (available from Tokyo Chemical Industry Co., Ltd.), 28.67 g of butyl methacrylate (available from Tokyo Chemical Industry Co., Ltd.), 0.70 g of 2,2'-azobis(2,4-dimethylvaleronitrile) (product name; V-65, available from Wako Pure Chemical Industries, Ltd.) and 110.84 g of ethanol while maintaining to 35°C or lower. Further, an aqueous solution of 0.70 g of 2,2'-azobis(N-(2-carboxyethyl)-2-methylpropionamidine) n-hydrate (product name of Wako Pure Chemical Industries, Ltd.; VA-057, available from Wako Pure Chemical Industries, Ltd.) dissolved in 27.71 g of pure water was added to the above-mentioned solution while maintaining to 35°C or lower, and the mixed solution into which all the above-mentioned materials were charged which became uniform by sufficient stirring, was introduced into a dropping funnel. On the other hand, 56.81 g of pure water and 131.62 g of ethanol were charged in a three-necked flask equipped with a cooling tube separately and subject to nitrogen flow, and heated to a reflux temperature while stirring. While maintaining the state, the dropping funnel into which the above-mentioned mixed solution was charged was set to the three-necked flask, and the mixed solution was added dropwise into a boiled liquid of pure water and ethanol over 1 hour. After the dropping, the mixture was stirred under heating for 24 hours while maintaining the above-mentioned circumstance. By cooling after 24 hours, 432.97 g of a copolymer-containing varnish having a solid content of about 19.86% by mass was obtained. A weight average molecular weight of the obtained colloidal state liquid by GFC was about 8,500.

### <Preparation Example 1>

To 10.00 g of the copolymer-containing varnish obtained in the above-mentioned Synthetic Example 1 were added 1.19 g of 1 mol/L hydrochloric acid (IN) (available from Wako KANTO CHEMICAL CO., INC.), 26.78 g of pure water and 62.54 g of ethanol and the mixture was sufficiently stirred to prepare a coating agent. The pH was 3.6.

### <Example 1>

The coating agent obtained in Preparation Example 1 was charged in 96-well microplate (manufactured by Asahi Glass Company, Code 5866-096) made of glass at the bottom portion with 0.3 mL/well and allowed to stand at 25°C for 1 hour. After removing the coating agent from the wells, these were dried at 25°C for 3 hours. Thereafter, by washing the wells with pure water sufficiently, a microplate made of glass at the bottom portion onto which a coating film has been formed was obtained.

### <Example 2>

The coating agent obtained in Preparation Example 1 was charged in 384-well microplate (manufactured by Greiner Bio-One Inc.) made of polystyrene (PS) with 0.1 mL/well and allowed to stand at 25°C for 1 hour. After removing the coating agent from the wells, these were dried at 25°C for 3 hours. Thereafter, by washing the wells with pure water sufficiently, a microplate made of PS at the bottom portion onto which a coating film has been formed was obtained.

### <Test Example 1>

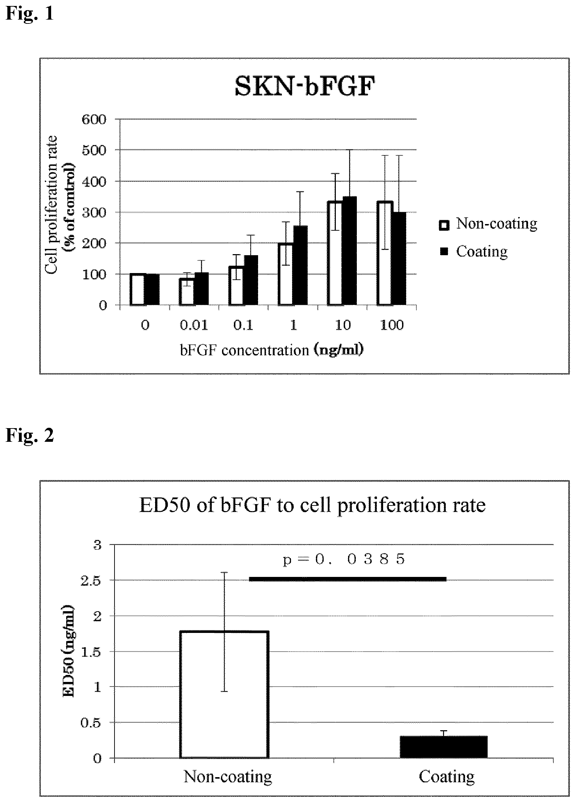

Uterine leiomyosarcoma cell line (SKN cells, JCRB cell bank) was seeded to a microplate made of glass at the bottom portion onto which a coating film obtained in Example 1 had been formed with 1,000 cells/0.1 mL/well using a Ham's F-12 medium (available from Wako Pure Chemical Industries, Ltd.) to which 0.015% (w/v) of deacylated gellan gum (KELCOGEL CG-LA, available from SANSHO Co., Ltd.) had been added in accordance with the method of WO2014/017513. As a control, SKN cells were similarly seeded to a microplate made of glass at the bottom portion onto which no coating film was formed. Also, to the seeded cells was added human bFGF (FGF-2: Human Recombinant, Basic fibroblast growth factor, available from ReproCELL INC.) so that the final concentration became 0, 0.01, 0.1, 1, 10 and 100 ng/mL, respectively, and culture was carried out under the conditions at 37°C and 5% CO₂ for 4 days. After the culture, the number of the cells was calculated by measuring the luminescence value of each well with a multimode microplate reader FlexStation 3 (manufactured by Molecular Devices LLC) using CellTiter-Glo (Registered Trademark) Luminescent Cell Viability Assay (manufactured by Promega Corporation). The results are shown in Fig. 1 and Fig. 2. Fig. 1 shows the calculation result of the cell proliferation rate at each bFGF concentration with the number of the cells when the bFGF is 0 ng/mL as 100%. Also, Fig. 2 shows Effective dose 50 (ED50) value of the bFGF concentration to the cell proliferation rate calculated based on the results of Fig. 1.

From Fig. 1 and Fig. 2, it could be confirmed that the cell proliferation rate of the SKN cells was increased by subjecting to coating, and the ED50 of the bFGF to the cell proliferation rate was significantly reduced.

### <Test Example 2>

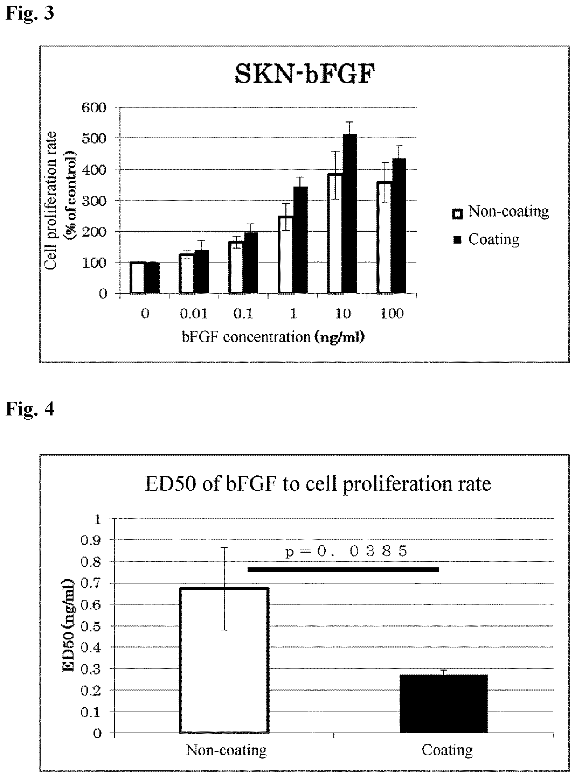

Uterine leiomyosarcoma cell line (SKN cells, JCRB cell bank) was seeded to a microplate made of PS onto which a coating film obtained in Example 2 had been formed with 450 cells/0.045 mL/well using a Ham's F-12 medium (available from Wako Pure Chemical Industries, Ltd.) to which 0.015% (w/v) of deacylated gellan gum (KELCOGEL CG-LA, available from SANSHO Co., Ltd.) had been added in accordance with the method of WO2014/017513. As a control, SKN cells were similarly seeded to a microplate made of PS onto which no coating film was formed. Also, to the seeded cells was added human bFGF (FGF-2: Human Recombinant, Basic fibroblast growth factor, available from ReproCELL INC.) so that the final concentration became 0, 0.01, 0.1, 1, 10 and 100 ng/mL, respectively, and culture was carried out under the conditions at 37°C and 5% CO₂ for 4 days. After the culture, the number of the cells was calculated by measuring the luminescence value of each well with a multimode microplate reader FlexStation 3 (manufactured by Molecular Devices LLC) using CellTiter-Glo (Registered Trademark) Luminescent Cell Viability Assay (manufactured by Promega Corporation). The results are shown in Fig. 3 and Fig. 4. Fig. 3 shows the calculation result of the cell proliferation rate at each bFGF concentration with the number of the cells when the bFGF is 0 ng/mL as 100%. Also, Fig. 4 shows Effective dose 50 (ED50) value of the bFGF concentration to the cell proliferation rate calculated based on the results of Fig. 3.

From Fig. 3 and Fig. 4, it could be confirmed that the cell proliferation rate of the SKN cells was increased by subjecting to coating, and the ED50 of the bFGF to the cell proliferation rate was significantly reduced.

### <Test Example 3>

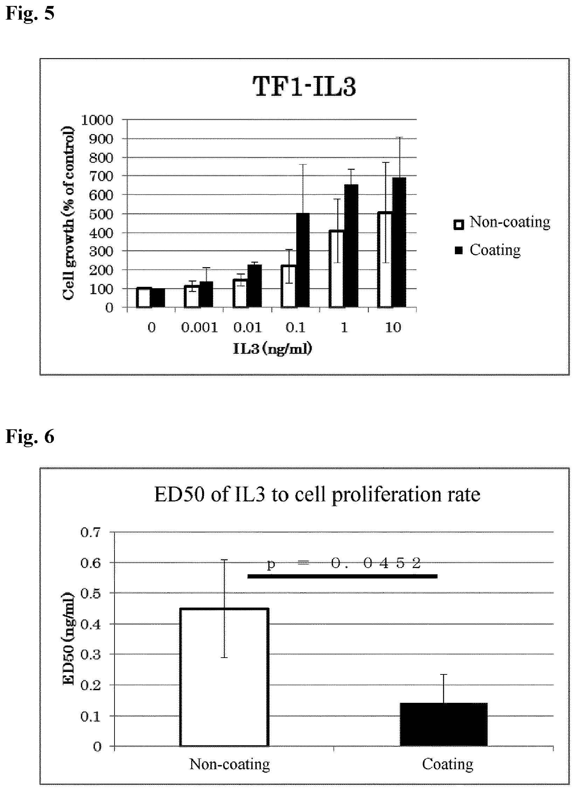

Human myelogenous leukemia cell (TF1 cells, Summit Pharmaceuticals International Corporation) was seeded to a microplate made of glass at the bottom portion onto which a coating film obtained in Example 1 had been formed with 1,000 cells/0.1 mL/well using an RPMI 1640 medium (available from Wako Pure Chemical Industries, Ltd.) to which sodium pyruvate (available from Wako Pure Chemical Industries, Ltd.) had been added. As a control, TF1 cells were similarly seeded to a microplate made of glass at the bottom portion onto which no coating film was formed. Also, to the seeded cells was added interleukin-3 (IL3, available from Wako Pure Chemical Industries, Ltd.) so that the final concentration became 0, 0.001, 0.01, 0.1, 1 and 10 ng/mL, respectively, and culture was carried out under the conditions at 37°C and 5% CO₂ for 4 days. After the culture, the number of the cells was calculated by measuring the luminescence value of each well with a multimode microplate reader FlexStation 3 (manufactured by Molecular Devices LLC) using CellTiter-Glo (Registered Trademark) Luminescent Cell Viability Assay (manufactured by Promega Corporation). The results are shown in Fig. 5 and Fig. 6. Fig. 5 shows the calculation result of the cell proliferation rate at each IL3 concentration with the number of the cells when the IL3 is 0 ng/mL as 100%. Also, Fig. 6 shows Effective dose 50 (ED50) value of the IL3 concentration to the cell proliferation rate calculated based on the results of Fig. 5.

From Fig. 5 and Fig. 6, it could be confirmed that the cell proliferation rate of the TF1 cells was increased by subjecting to coating, and the ED50 of the IL3 to the cell proliferation rate was significantly reduced.

### <Test Example 4>

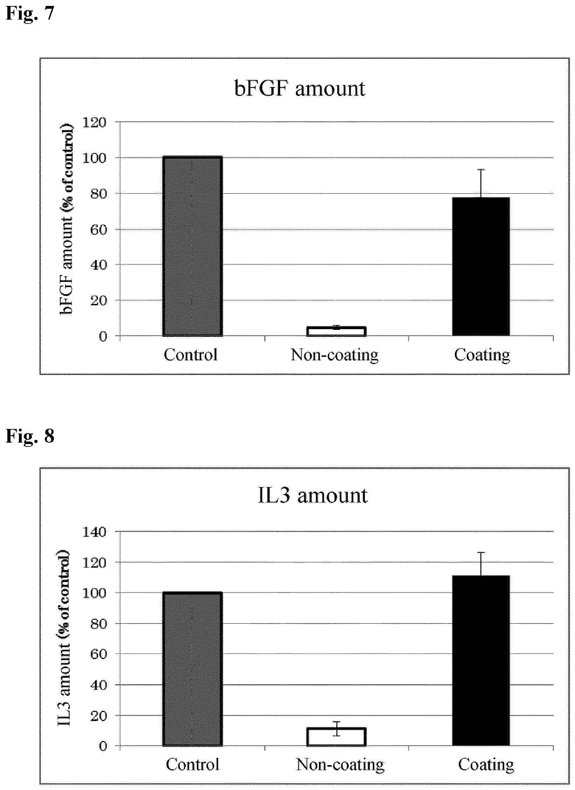

A Ham's F-12 medium (Wako Pure Chemical Industries, Ltd.) to which bFGF (available from Wako Pure Chemical Industries, Ltd.) had been added was charged in a microplate made of glass at the bottom portion onto which a coating film obtained in Example 1 with 0.1 mL/well. As a control, a Ham's F-12 medium to which bFGF had been added was similarly charged into a microplate made of glass at the bottom portion onto which no coating film was formed. After subjecting to incubation at 37°C for 6 hours, the bFGF amount in the Ham's F-12 medium was determined in accordance with the protocol using a bFGF ELISA kit (manufactured by Raybiotech, Inc.). Fig. 7 shows the bFGF amount in the Ham's F-12 medium after subjecting to incubation when the bFGF amount in the Ham's F-12 medium subjecting to no incubation is made 100%.

From Fig. 7, it was shown that adsorption of the bFGF could be inhibited by subjecting to coating.

### <Test Example 5>

An RPMI 1640 medium (Wako Pure Chemical Industries, Ltd.) to which IL3 (available from ReproCELL INC.) had been added was charged into a microplate made of glass at the bottom portion onto which a coating film obtained in Example 1 with 0.1 mL/well. As a control, an RPMI 1640 medium to which IL3 had been added was similarly charged into a microplate made of glass at the bottom portion onto which no coating film was formed. After subjecting to incubation at 37°C for 4 days, the IL3 amount in the RPMI 1640 medium was determined in accordance with the protocol using an IL3 ELISA kit (manufactured by Abcam plc.). Fig. 8 shows the IL3 amount in the RPMI 1640 medium after subjecting to incubation when the IL3 amount in the RPMI 1640 medium subjecting to no incubation is made 100%.

From Fig. 8, it was shown that adsorption of the IL3 could be inhibited by subjecting to coating.

### <Test Example 6>

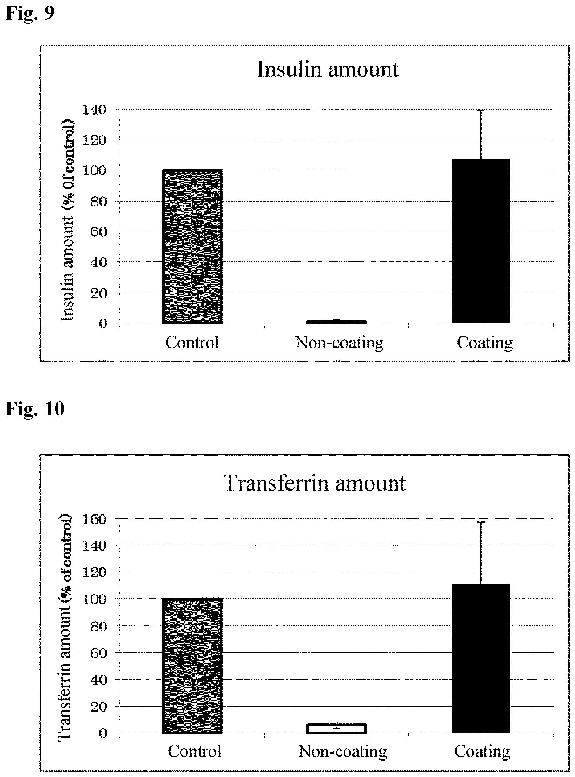

A Dulbecco's Modified Eagle's Medium (DMEM, available from Wako Pure Chemical Industries, Ltd.) to which an insulin-transferrin-selenium aqueous solution (ITS aqueous solution, available from Thermo Fisher Scientific Inc.) had been added was charged into a microplate made of glass at the bottom portion onto which a coating film obtained in Example 1 with 0.1 mL/well. As a control, a DMEM to which an ITS aqueous solution had been added was similarly charged into a microplate made of glass at the bottom portion onto which no coating film was formed. After subjecting to incubation at 37°C for 3 days, the insulin amount and the transferrin amount in the DMEM were determined in accordance with the protocol by using an insulin ELISA kit (manufactured by Mercodia AB) and a transferrin ELISA kit (manufactured by Abcam plc.), respectively. Fig. 9 shows the insulin amount in the DMEM after subjecting to incubation when the transferrin amount in the DMEM subjecting to no incubation is made 100%, and Fig. 10 shows the transferrin amount in the DMEM after subjecting to incubation when the insulin amount in the DMEM subjecting to no incubation is made 100%, respectively.

From Fig. 9 and Fig. 10, it was shown that adsorption of the insulin and transferrin could be inhibited by subjecting to coating.

### <Example 3>

The coating agent obtained in Preparation Example 1 was charged into a 96-well microplate (manufactured by Corning Inc., #3595) made of PS with 0.3 mL/well, and allowed to stand at 25°C for 1 hour. After moving the coating agent, these were dried at 25°C for 3 hours. Thereafter, by washing the wells with pure water sufficiently, a 96-well microplate made of PS onto which a coating film has been formed was obtained.

### <Test Example 7>

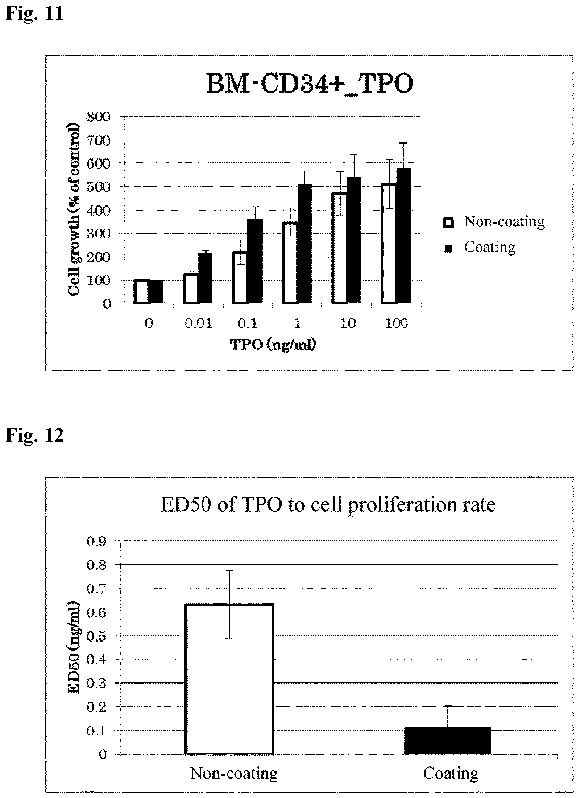

Human bone marrow-derived CD34 positive progenitor cells (Bone Marrow CD34+ cells (BM-CD34+), LONZA Co., Product Code 2M-101B) were seeded to a 96-well microplate made of PS onto which a coating film obtained in Example 3 had been formed with 2,000 cells/0.1 mL/well using a StemSpan™SFEM medium (available from STEMCELL TECHNOLOGIES Inc., CATALOG #09650). As a control, BM-CD34+ cells were similarly seeded to a 96-well microplate made of PS onto which no coating film was formed. Also, to the seeded cells were added Stem Cell Factor (SCF, available from PEPROTECH, Inc., Catalog Number: 300-07) so that the final concentration became 100 ng/mL and further Thrombopoietin (TPO, available from PEPROTECH, Inc., Catalog Number: 300-18) so that the final concentration became 0, 0.01, 0.01, 0.1, 1, 10 and 100 ng/mL, respectively, and culture was carried out under the conditions at 37°C and 5% CO₂ for 7 days. After the culture, the number of the cells was calculated by measuring the luminescence value of each well with a multimode microplate reader FlexStation 3 (manufactured by Molecular Devices LLC) using CellTiter-Glo (Registered Trademark) Luminescent Cell Viability Assay (manufactured by Promega Corporation). The results are shown in Fig. 11 and Fig. 12. Fig. 11 shows the calculation result of the cell proliferation rate at each TPO concentration with the number of the cells when TPO is 0 ng/mL as 100%. Also, Fig. 12 shows Effective dose 50 (ED50) value of the TPO concentration to the cell proliferation rate calculated based on the results of Fig. 11.

From Fig. 11 and Fig. 12, it could be confirmed that the cell proliferation rate of the BM-CD34+ cells was increased by subjecting to coating, and the ED50 of the TPO to the cell proliferation rate was significantly reduced.

### <Test Example 8>

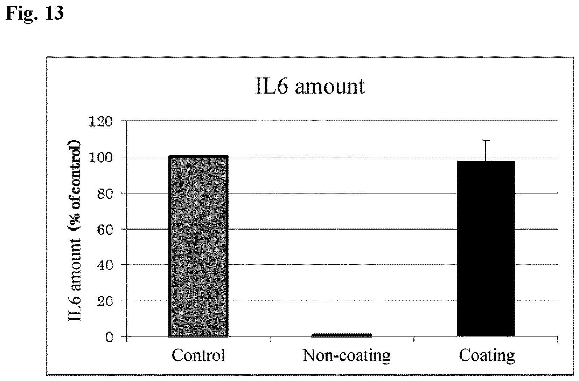

An RPMI 1640 medium (Wako Pure Chemical Industries, Ltd.) to which interleukin-6 (IL6, available from ReproCELL INC.) had been added was charged into a microplate made of PS onto which a coating film obtained in Example 1 with 0.1 mL/well. As a control, an RPMI 1640 medium to which IL6 had been added was similarly charged into a microplate made of PS onto which no coating film was formed. After subjecting to incubation at 37°C for 4 days, the IL6 amount in the RPMI 1640 medium was determined in accordance with the protocol using an IL6 ELISA kit (manufactured by Abcam plc.). Fig. 13 shows the IL6 amount in the RPMI 1640 medium after subjecting to incubation when the IL6 amount in the RPMI 1640 medium subjecting to no incubation is made 100%.

From Fig. 13, it was shown that adsorption of the IL6 could be inhibited by subjecting to coating.

### UTILIZABILITY IN INDUSTRY

The cell culture vessel of the present invention can inhibit adhesion of not only cells but also peptides relating to cell proliferation by having a coating which inhibits adhesion of the peptide relating to cell proliferation, in particular, a coating containing a copolymer which contains an anionic group represented by the formula (a) and a cationic group represented by the formula (b), and if necessary, a hydrophobic group represented by the formula (c), at least a part of the surface of the vessel. Therefore, proliferation and function expression of the cells are not impaired, a proliferation rate of the cells in the cell culture can be increased, and an amount of the peptide relating to cell proliferation to be added can be reduced. Also, the coating provided by the cell culture vessel of the present invention has good adhesiveness to a resin such as plastic, etc., and can be easily formed onto at least a part of the surface of the vessel. Further, the above-mentioned coating is excellent in resistance to an aqueous solvent, so that elution of the copolymerized component from the coating into the culture medium is suppressed, whereby no bad effect is exerted to cells or peptides relating to cell proliferation and the problem such as residue thereof in the final products can be also solved.

## Claims

1. A cell culture vessel which comprises a coating which inhibits adhesion of a peptide relating to cell proliferation at least a part of a surface thereof.

2. The cell culture vessel according to Claim 1, wherein the peptide relating to cell proliferation is a peptide relating to cell proliferation through a receptor or a signal, or a peptide relating to nutrient transportation.

3. The cell culture vessel according to Claim 2, wherein the peptide relating to cell proliferation through a receptor or a signal is selected from the group consisting of a cell growth factor, a hematopoietic factor, a neurotrophic factor, interleukins and hormones.

4. The cell culture vessel according to Claim 1, wherein the coating contains a copolymer containing a recurring unit which contains a group represented by the following formula (a) and a recurring unit which contains a group represented by the following formula (b): 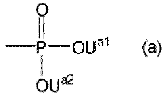 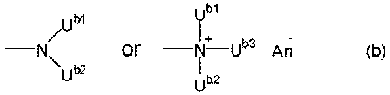 wherein,
U^{a1}, U^{a2}, U^{b1}, U^{b2} and U^{b3} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion.

5. The cell culture vessel according to Claim 4, wherein the copolymer further contains a recurring unit which contains a group represented by the following formula (c):
-R^{c} (c)
wherein,
R^{c} represents a linear or branched alkyl group having 1 to 18 carbon atoms, an alicyclic hydrocarbon group having 3 to 10 carbon atoms, an aryl group having 6 to 10 carbon atoms, an aralkyl group having 7 to 15 carbon atoms or an aryloxyalkyl group having 7 to 15 carbon atoms, where, the above-mentioned aryl portion may be substituted by a linear or branched alkyl group having 1 to 5 carbon atoms which may be substituted by a halogen atom(s).

6. The cell culture vessel according to Claim 3, wherein the cell growth factor is selected from the group consisting of an epidermal growth factor (EGF), an insulin-like growth factor (IGF), a transforming growth factor-β (TGF-β), a nerve growth factor (NGF), a brain-derived neurotrophic factor (BDNF), a vascular endothelial cell growth factor (VEGF), a hepatocyte growth factor (HGF), a platelet-derived growth factor (PDGF), an acidic fibroblast cell growth factor (aFGF) and a basic fibroblast cell growth factor (bFGF).

7. The cell culture vessel according to Claim 3, wherein the a hematopoietic factor is selected from the group consisting of a granulocyte-colony stimulating factor (G-CSF), a granulocyte-macrophage colony-stimulating factor (GM-CSF), a macrophage colony-stimulating factor (M-CSF), a monocyte chemotactic factor (MCP-1), erythropoietin (EPO) and thrombopoietin (TPO).

8. The cell culture vessel according to Claim 3, wherein the interleukin is selected from the group consisting of interleukin-la, interleukin-1β, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18 and leukemia inhibitory factor (LIF).

9. The cell culture vessel according to Claim 3, wherein the neurotrophic factor is selected from the group consisting of nerve growth factor (NGF), brain-derived neurotrophic factor (BDNF), neurotrophin 3 and neurotrophin 4/5.

10. The cell culture vessel according to Claim 3, wherein the hormone is selected from the group consisting of insulin, glucagon, adrenocorticotropic hormones, thyroid stimulating hormones, growth hormones, follicle stimulating hormones, luteotropic hormones, prolactin and fibronectin.

11. The cell culture vessel according to Claim 2, wherein the peptide relating to nutrient transportation is transferrin or bovine serum albumin (BSA).

12. A method for increasing a proliferation rate of cells cultured in a cell culture vessel which comprises subjecting a coating which inhibits adhesion of a peptide relating to cell proliferation to at least a part of a surface of the vessel.

13. A method for reducing an amount of a peptide relating to cell proliferation to be added to a cell culture vessel which comprises subjecting a coating which inhibits adhesion of a peptide relating to cell proliferation to at least a part of a surface of the vessel.
